# EUROPEAN PATENT SPECIFICATION

(11) **EP 3 092 222 B1**
(45) Date of publication and mention of the grant of the patent: **14.02.2018**
(21) Application number: 14859350.2
(22) Date of filing: 24.12.2014
(51) Int. Cl.: C07C 213/00, C07C 215/28, C07C 45/30, C07C 47/228, C07D 319/06

(54) **IMPROVED FINGOLIMOD PROCESS**
VERBESSERTES VERFAHREN FÜR FINGOLIMOD
PROCÉDÉ DE PRÉPARATION DE FINGOLIMOD AMÉLIORÉ

(30) Priority: 07.01.2014 IN 53MU2014
(43) Date of publication of application: 16.11.2016
(73) Proprietor: Emcure Pharmaceuticals Limited, Pune 411026 (IN)
(72) Inventor: GURJAR, Mukund Keshav, Pune 411026 Maharashtra (IN); TRIPATHY, Narendra Kumar, Pune 411026 Maharashtra (IN); PRAMANIK, Chinmoy Mriganka, Pune 411026 Maharashtra (IN); CHAUGULE, Balaji Vasant, Pune 411026 Maharashtra (IN); KARHADE, Ganesh Kaluram, Pune 411026 Maharashtra (IN); BORHADE, Ajit Sahebrao, Pune 411026 Maharashtra (IN); PATOLE, Jayendra Dattatraya, Pune 411026 Maharashtra (IN); NEELAKANDAN, Kaliyaperumal, Pune 411026 Maharashtra (IN); MEHTA, Samit Satish, Pune 411026 Maharashtra (IN)
(74) Representative: Engelhard, Markus
(86) International application number: PCT/IN2014/000797
(87) International publication number: WO 2015/107548

(56) References cited:
- EP-A2- 0 348 223
- WO-A2-2012/056458
- CN-A- 102 731 324

## Description

### FIELD OF THE INVENTION

The present invention relates to a novel and cost-effective method for preparation of fingolimod (I) conforming to regulatory specifications. Specifically, the method relates to a synthetic route that involves isolation of solid intermediates having impurity levels below regulatory limits by employing 5-nitro-2,3-dimethyl-1,3-dioxane of formula (7), which subsequently results in providing Fingolimod hydrochloride conforming to regulatory specifications.

### BACKGROUND OF THE INVENTION

Fingolimod (I) administered as its hydrochloride salt (Ia) and chemically known as 2-amino-2-(2-(4-octylphenyl)ethyl)-propan-1,3-diol is a sphingosine 1-phosphate receptor modulator indicated for the treatment of patients with relapsing forms of multiple sclerosis for reducing the frequency of clinical exacerbations and to delay the accumulation of physical disability. Fingolimod hydrochloride capsule with proprietary name 'GILENYA' and strength of 0.50 mg was approved by USFDA on September 21, 2010 for oral administration.

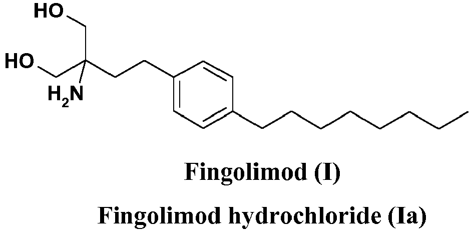

Various researchers have attempted to synthesize fingolimod (I) and its hydrochloride salt (Ia). However, these processes are associated with formation of impurities such as dimer impurity and those arising from polymerization of paraformaldehyde during nitro-aldol condensation, wherein the extent of impurity formation is above regulatory limits. Accordingly, the prior art processes require several steps of purifications involving column chromatography for removal of the impurities from the said intermediates as well as from the final product.

CN 1765872 discloses a process for fingolimod by nitration of p-octyl-3-bromopropiophenone with sodium nitrite to give 3-nitro-1-(4-octylphenyl) propane-1-one with a low yield, which is possibly due to the formation of the side product, O-alkyl (R-O-NO). Subsequent reduction of the ketone followed by nitro-aldol condensation with formaldehyde results in 2-nitro-2-[2-(4-octylphenyl)-2-hydroxyethyl]-1,3-propanediol which is obtained as an oily compound and is further reduced to give fingolimod with an overall yield of 56% which is not economical for commercial scale.

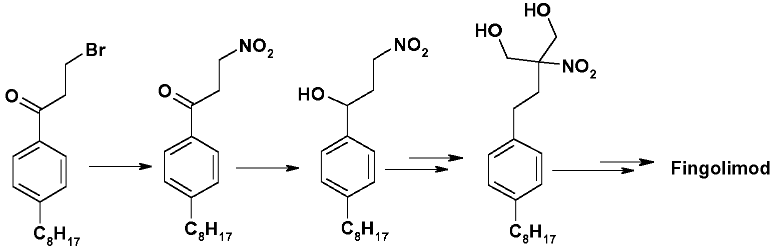

In the above synthetic strategy, the 3-nitro-1-(4-octylphenyl)propanol intermediate is susceptible to dehydration (under heat or acid / base catalyst) to form the corresponding undesired impurity of nitro alkene of formula (2), which may undergo isomerization, decomposition or polymerization to form further undesired impurities.

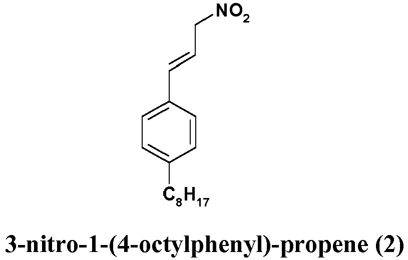

CN 1310869, WO2012041405, WO2012041358 and Synlett 2001, (9)1411-1414, discloses a method for preparing 2-nitro-2-[2-(4-octylphenyl)-ethyl]-1,3-propanediol by treatment of 4-octylphenyl-3-nitropropane with paraformaldehyde, followed by reduction of the nitro group to give fingolimod.

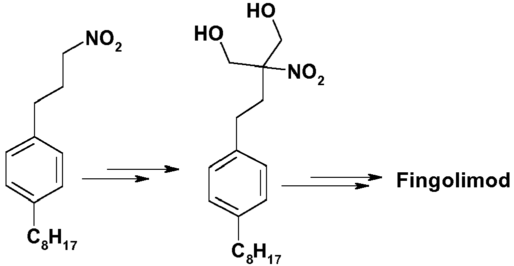

The above prior art methods utilize formaldehyde or paraformaldehyde for conversion of 4-octylphenyl-3-nitropropane and 3-nitro-1-(4-octylphenyl)propanol intermediates. However, these methods suffer from an inherent disadvantage as formaldehyde polymerizes during nitro-aldol reaction, which results in a very low yield. Further it was also found that replication of the method disclosed in CN 1765872 involving reduction of 3-nitro-1(4-octylphenyl) propane-1-one with a reducing agent, resulted in formation of 2-nitro-2-[2-(4-octylphenyl)-2-hydroxylethyl]-1,3-propanediol which finally resulted in formation of the impurity, 1-(4-octylphenyl)-propanol of formula (3).

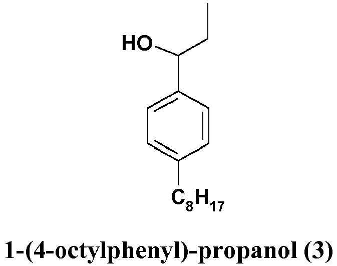

WO 2012056458 discloses various alternate methods for fingolimod. In one of the methods, the keto group of 1-(4-(3-nitropropyl)phenyl)octan-1-one, during reaction with formaldehyde, is likely to undergo nitro-aldol condensation with another aldehyde molecule to form a dimeric impurity, which is very difficult to remove from the intermediate or the final product. This route of synthesis also involves multiple steps for preparation of fingolimod, which increases the cost of process, effluents and imposes additional burden on the environment.

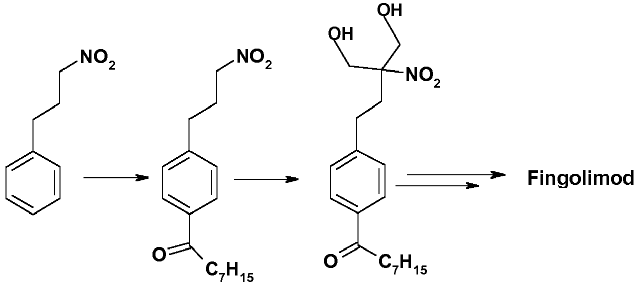

The other method involves Friedel-Craft reaction of 2-dimethyl-5-nitro-5-phenethyl-1,3-dioxane with octanoyl chloride to provide 1-(4-(2,2-dimethyl-5-nitro-1,3-dioxan-5-yl)ethyl)phenyl)octan-1-one. The presence of acidic conditions due to presence of Lewis acid is likely to result in deprotection of the 1,3-dioxane ring in the starting material to give 2-nitro-(2-phenyl)ethyl]-1,3-propanediol as an associated impurity.

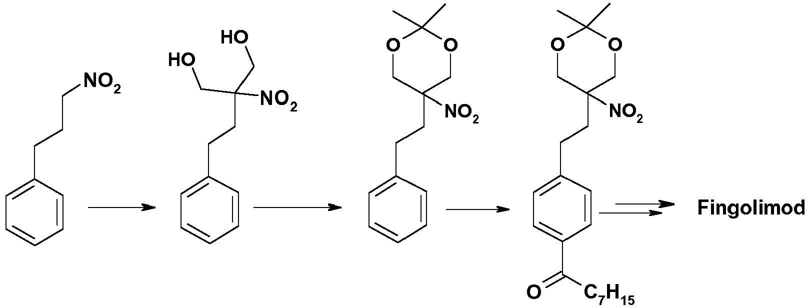

In yet another method, the use of phosphine and phosphonate intermediates makes the process less suitable and unviable for regulated markets due to the strict guidelines on the genotoxic impurities generated during the coupling reaction in the intermediate and final stages of preparation of fingolimod. Additionally, the amino protected intermediate, 1-(4-(2,2-dimethyl-5-amino-1,3-dioxan-5-yl)etheneyl)phenyl)octan-1-one of formula (4) has a tendency to undergo self condensation to form the dimer impurity.

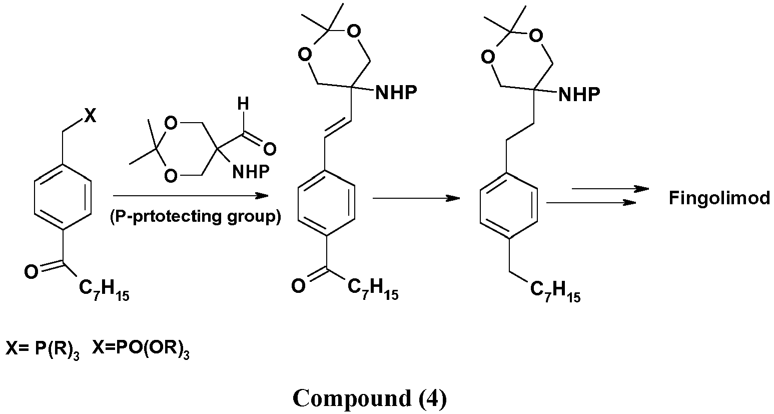

EP 2502901 discloses an alternative route of synthesis for fingolimod by utilizing 4-octyl benzyl bromide and mercapto benzothiazole and subsequent reaction with CBZ protected 5-amino-5-formyl-2-dimethyl-1,3-dioxane, followed by hydrolysis to obtain fingolimod. The mercapto benzothiazole and CBZ protected 5-amino-5-formyl-2-dimethyl-1,3-dioxane being hazardous, poses severe limitations for their use on the industrial scale. Further, the method also involves additional steps of protection and deprotection of the amino and hydroxyl group thereby, making the method lengthy and less attractive for industrial scale.

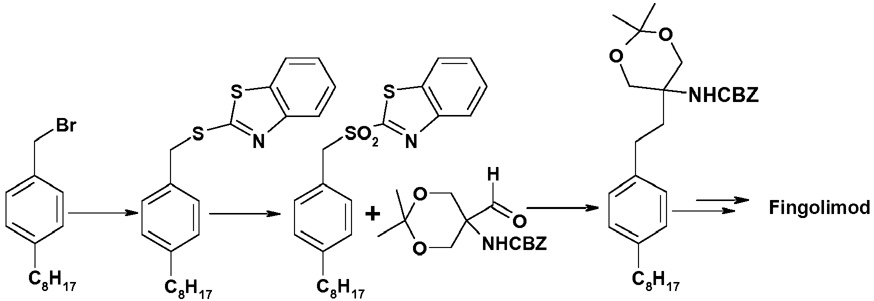

CN 1483721 (CN 1212308C) provides a process for preparation of fingolimod by reduction of octanoyl benzene to octyl benzene, followed by reaction with halogenated acetyl halide or halogenated acetic anhydride in presence of a catalyst to give 4-haloacetyloxyoctyl benzene, which on subsequent reduction gives 4-(2-haloethyl)octyl benzene. Further, condensation with diethylmalonate under alkaline conditions and nitrosation results in 2-nitro-(2-(4-octylphenyl) ethyl)diethylmalonate, which was then subjected to reduction to give fingolimod. Herein, the major drawback is the use of lithium aluminium hydride (LAH) which results in lower yield as it is very difficult to isolate product from the reaction mixture and hence the method is not suitable for industrial scale.

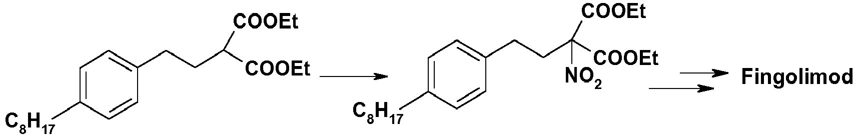

US 5604229, WO20012041405 (WO2012041707) and WO2012041359 provides yet another process for preparation of fingolimod wherein the key intermediate, diethyl-2-acetamido-2-(4-octyl phenyl)ethyl malonate was prepared by reaction of 2-(4-octylphenyl)ethyl iodide with diethyl acetamido malonate in presence of a base.

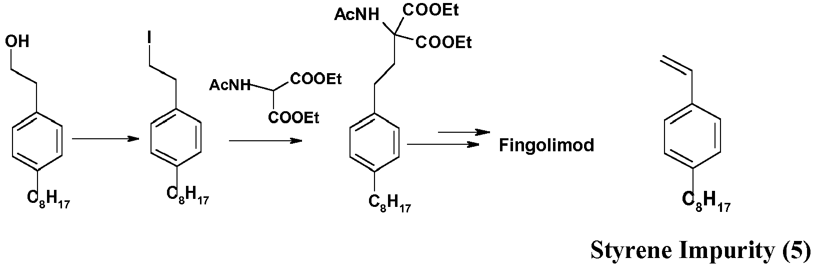

This method is associated with formation of the styrene impurity (5) during iodination reaction and although this impurity does not present separation related problems, it considerably affects the yield of the final product.

It would be evident from the foregoing that prior art methods are associated with tedious and circuitous synthetic routes and produce intermediates as oily substances or various isomeric mixtures which are also associated with impurities of formula (2), (3) and (5) as well as dimer impurity. Thus the elimination of these impurities either by column chromatographic purification or by successive crystallizations is inevitable, results in loss in yield and ultimately makes the process unviable for commercial use.

Regulatory authorities all over the world have very stringent norms for permissible limits of such impurities, either in the active ingredient or in drug product. Thus, there is a need to develop a new synthetic route which makes it possible to prepare the desired product with purity conforming to regulatory limits and is suitable for commercial scale production.

Thus, it was imperative to control such impurities below detectable limits during synthesis and develop a route involving a cost-effective process which did not require additional steps of purification. Hence, to overcome the prior art drawbacks, the present inventors developed a new synthetic route for fingolimod which involves lesser number of synthetic steps, controls the level of undesired impurities below permissible limits and is cost-effective, environmental friendly and convenient for implementation on an industrial scale.

The present invention provides a new synthetic route for fingolimod wherein, the method does not require stringent, hazardous reaction conditions and avoids reagents such as lithium aluminum hydride, nitrating agents like sodium nitrite, nitric acid. Further, a significant advantage of this synthetic method is that all the intermediates can be isolated as crystalline solids as compared to prior art wherein the intermediates are mostly obtained as oil or sticky solid. It should be noted that during the isolation, the associated impurities get filtered into the mother liquor due to which the solid intermediates are free from associated impurities and thus help in providing fingolimod hydrochloride of desired purity.

### OBJECTS OF THE INVENTION

The main object of the invention is to provide an industrially viable method for preparing fingolimod and its pharmaceutically acceptable salts, conforming to regulatory specification.

Another object of the present invention is to provide a novel synthetic route for preparation of fingolimod and its pharmaceutically acceptable salts, which circumvents the isolation of associated impurities by obtaining the intermediates in a crystalline solid form as compared to prior art in which the intermediates are frequently obtained as an oil due to the presence of impurities, which are difficult to be removed.

### SUMMARY OF THE INVENTION

The main aspect of the present invention is to provide a convenient synthetic route for the preparation of fingolimod and its pharmaceutically acceptable salts conforming to regulatory specification.

Another aspect of the present invention is to provide a method for preparation of fingolimod comprising,
1. A process for preparation of fingolimod hydrochloride comprising,
   a) reacting 2-(4-octylphenyl)-ethanol with an oxidizing agent, in an organic solvent to give 2-(4-octylphenyl)-acetaldehyde of formula (6),
   b) treating compound of formula (6) with a 5-nitro-2,3-dimethyl-1,3-dioxane of formula (7) in presence of a base to give 1-(4-(2-hydroxy-2-(2,2-dimethyl-5-nitro-1,3-dioxane-5-yl)ethyl) phenyl) octane of formula (8),
   c) treating compound of formula (8) with substituted sulfonyl chloride in presence of triethylamine to give the sulfonates of compound (9),
   d) treating compound of formula (9) with a base to give 1-(4-(2-(2,2-dimethyl-5-nitro-1,3-dioxane-5-yl)-ethene-1-yl)phenyl)octane of formula (10), which on hydrogenation gives 1-(4-(2-(2,2-dimethyl-5-amino-1,3-dioxane-5-yl)-ethyl)phenyl)octane of formula (11),
   e) treating compound of formula (11) with an acid to yield fingolimod (I) which is further converted to its hydrochloride salt.

Yet another aspect of the invention comprises,
a) reacting 2-(4-octylphenyl)-ethanol with an oxidizing agent, in an organic solvent to give 2-(4-octylphenyl)-acetaldehyde of formula (6),
b) treating compound of formula (6) with 5-nitro-2,3-dimethyl-1,3-dioxane of formula (7) in presence of a base and in an organic solvent to give 1-(4-(2-hydroxy-2-(2,2-dimethyl-5-nitro-1,3-dioxane-5-yl)ethyl)phenyl)octane of formula (8),
c) treating compound of formula (8) with a dehydrating agent to give 1-(4-(2-(2,2-dimethyl-5-nitro-1,3-dioxane-5-yl)-ethene-1-yl)phenyl)octane of formula (10),
d) hydrogenating compound of formula (10) to give 1-(4-(2-(2,2-dimethyl-5-amino-1,3-dioxane-5-yl)ethyl)phenyl)octane of formula (11),
e) treating compound of formula (11) with an acid to yield fingolimod (I) which is further converted to its hydrochloride salt.

### DETAILED DESCRIPTION OF THE INVENTION

The present invention as disclosed in Scheme-I relates to a method in which the intermediates are obtained in the solid form due to their high purity and with impurity levels conforming to regulatory limits.

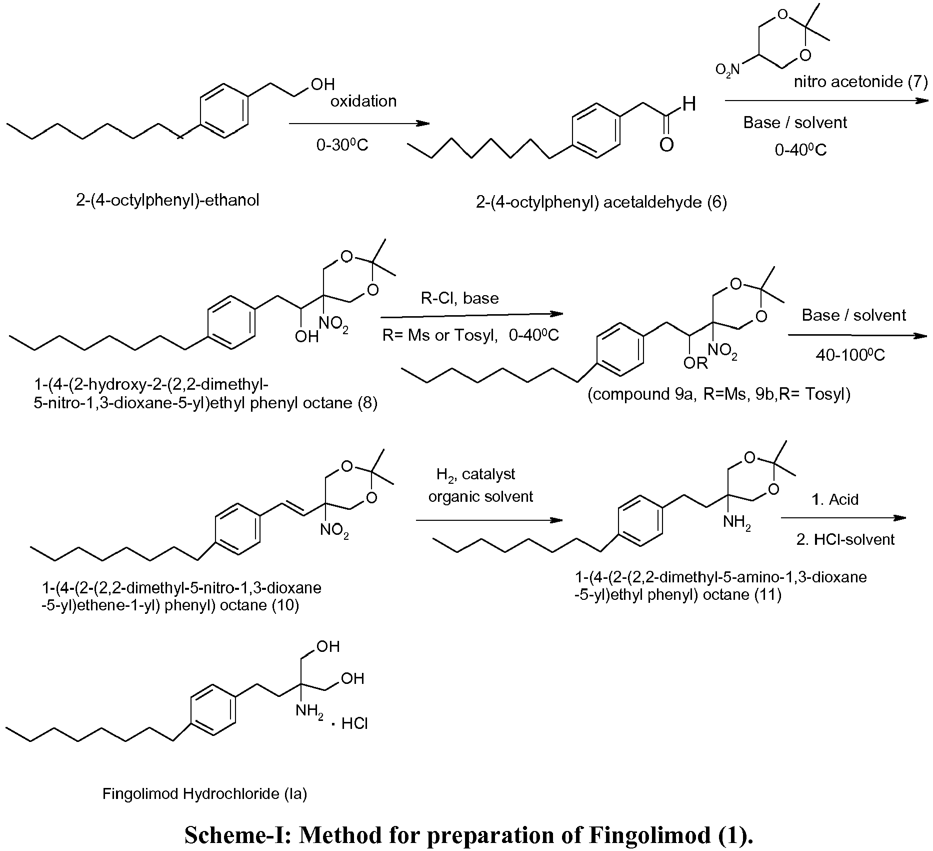

The method of the present invention involves oxidation of 2-(4-octylphenyl)-ethanol, which is obtained by prior art methods.

Oxidation of 2-(4-octylphenyl)-ethanol is carried out with an oxidizing agent in an organic solvent to give 2-(4-octylphenyl)-acetaldehyde (6).

The oxidizing agent is selected from the group comprising of Dess-Martin Periodinane (DMP), (2,2,6,6-Tetramethylpiperidin-1-yl)oxidanyl (TEMPO), Sodium hypochlorite, Pyridinium dichromate (PDC), Pyridinium chlorochromate (PCC), Dimethylsulfoxide/triethylamine/oxalyl chloride (DMSO/TEA/(COCl)₂, sulfur trioxide /pyridine (SO₃-Py). The organic solvent was selected from the group comprising of esters, ethers, nitriles, halogenated hydrocarbons and aromatic and aliphatic hydrocarbons.

The amount of oxidizing agent employed was in the range of 0.99 to 1.50 moles per mole of 2-(4-octylphenyl)-ethanol and the oxidation was carried out at a temperature of 0 to 30°C, preferably between 0 and 5°C.

The 2-(4-octylphenyl) acetaldehyde (6) thus obtained was treated with 5-nitro-2,3-dimethyl-1,3-dioxane (compound 7) in presence of a base and an organic solvent gave 1-(4-(2-hydroxy-2-(2,2-dimethyl-5-nitro-1,3-dioxane-5-yl)-ethyl)phenyl) octane (8).

The base selected was an organic or inorganic base. The organic base was selected from the group comprising of triethylamine (TEA), 1,8-Diazabicyclo[5.4.0]undec-7-ene (DBU), 1,5-Diazabicyclo(4.3.0)non-5-ene (DBN), 1,4-diazabicyclo[2.2.2]octane (DABCO), 2,6-dimethylpyridine (2,6-lutidine), diisopropylethylamine (DIPEA), N-methyl morpholine (NMM), pyridine, N,N-dimethylaminopyridine (DMAP), while the inorganic base was selected from group comprising of carbonates, bicarbonates, hydroxide, alkoxides of alkali metals such as sodium, potassium, lithium and active basic aluminium oxide.

The solvent was selected from the group of alcohols, esters, nitriles, halogenated hydrocarbons, and aromatic hydrocarbons. The solvent employed was selected from the group of methanol, ethanol, isopropanol, ethyl acetate, propyl acetate, isopropyl acetate, acetonitrile, dichloromethane, dichloroethane, chloroform, toluene, xylene and mixtures thereof.

The reaction was carried out at a temperature ranging from 0°C to 40°C, till completion of reaction, as monitored by HPLC. The organic layer was separated and concentrated. The residue was triturated with a hydrocarbon like cyclohexane.

1-(4-(2-Hydroxy-2-(2,2-dimethyl-5-nitro-1,3-dioxane-5-yl)-ethyl)phenyl) octane (8) was then treated with a substituted sulfonyl chloride as a hydroxyl protecting agent to give the protected intermediate (compound 9). In an embodiment, the compound 8 was treated with either methanesulfonyl chloride or p-toluene sulfonyl chloride in an organic solvent and in presence of an organic base like triethyl amine to give either 1-(4-(2-methanesulfonyl-2-(2,2-dimethyl-5-nitro-1,3-dioxane-5-yl)-ethyl)phenyl)octane (9a) or 1-(4-(2-p-tolylsulfonyl-2-(2,2-dimethyl-5-nitro-1,3-dioxane-5-yl)-ethyl)phenyl)octane (9b).

The reaction was carried out in a solvent selected from the group of aliphatic hydrocarbons, aromatic hydrocarbons, chlorinated hydrocarbons.

The reaction was carried out at a temperature ranging from 0°C to 40°C, to give the hydroxy protected intermediate. After completion of reaction as monitored by TLC, the organic layer was washed, dried and concentrated to a brown solid with yield of 85 to 90%.

1-(4-(2-Methanesulfonyl-2-(2,2-dimethyl-5-nitro-1,3 -dioxane-5-yl)-ethyl)phenyl) octane (9a) or 1-(4-(2-p-Tolylsulfonyl-2-(2,2-dimethyl-5-nitro-1,3-dioxane-5-yl)ethyl)phenyl)octane (9b) was treated with a base selected from 1,8-diazabicyclo[5.4.0]undec-7-ene (DBU), triethylamine(TEA), 1,5-Diazabicyclo(4.3.0)non-5-ene (DBN), 1,4-diazabicyclo[2.2.2]octane (DABCO), 2,6-dimethylpyridine (2,6-lutidine), diisopropylethylamine (DIPEA), N-methyl morpholine (NMM), pyridine, N,N-dimethylaminopyridine (DMAP) or mixtures thereof in presence of an organic solvent at temperature ranging from 40°C to 100°C to give 1-(4-(2-(2,2-dimethyl-5-nitro-1,3-dioxane-5-yl)-ethene-1-yl)phenyl)-octane (10).

The amount of base employed was in the range of 1.0-1.5 equivalents, but preferably between 1.1 and 1.3 equivalents per mole of the substrate (9a) or (9b).

The organic solvent was selected from the group comprising of aromatic hydrocarbon (toluene, xylene), aliphatic hydrocarbon (hexane, cyclohexane, heptane), aliphatic halogenated hydrocarbon (dichloromethane), amide (dimethylformamide) and sulfoxide (dimethyl sulphoxide). The solvent was preferably toluene or dichloromethane.

After completion of the reaction as monitored by HPLC, the reaction mixture was cooled and diluted with the same solvent. The organic layer was washed with aqueous acid and concentrated to get desired compound (10) with yield of 95%.

In yet another embodiment of the present invention, as represented in Scheme-II, 1-(4-(2-hydroxy-2-(2,2-dimethyl-5-nitro-1,3-dioxane-5-yl)-ethyl)phenyl)octane (8) was subjected to reaction with a dehydrating agent in presence of an organic solvent to give 1-(4-(2-(2,2-dimethyl-5-nitro-1,3-dioxane-5-yl)-ethene-1-yl)phenyl)octane of formula (10).

The dehydrating agent was selected from the group comprising of phosphorus pentoxide (P₂O₅), p-toluene sulfonic acid (PTSA) or mixtures thereof.

The organic solvent was selected from the group comprising of aromatic hydrocarbons such as toluene, xylene, chlorobenzene.

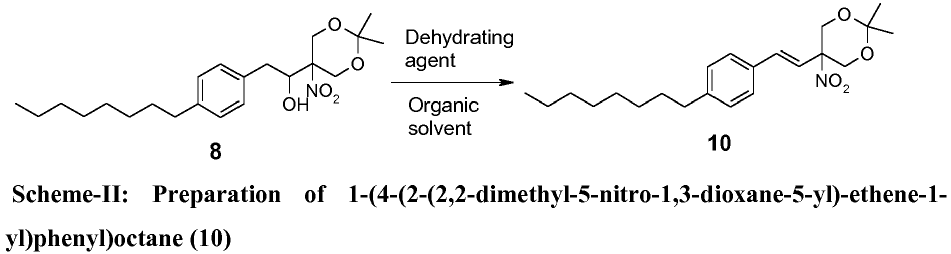

Catalytic hydrogenation of 1-(4-(2-(2,2-dimethyl-5-nitro-1,3-dioxane-5-yl)-ethene-1-yl)phenyl)-octane (10) in an organic solvent at temperature ranging from 50 to 95°C gave 1-(4-(2-(2,2-dimethyl-5-amino-1,3-dioxane-5-yl)-ethyl)phenyl)octane (11).

The catalyst was selected from the group comprising palladium on carbon, platinum on carbon Raney nickel.

Hydrogen pressure for the reaction was in the range of 2 to 15 kg /cm², but preferably 6-10 kg /cm².

The organic solvent was selected from the group of alcohols, esters or mixtures thereof. Preferred solvents were selected from methanol, ethanol, isopropanol, ethyl acetate, propyl acetate, isopropyl acetate.

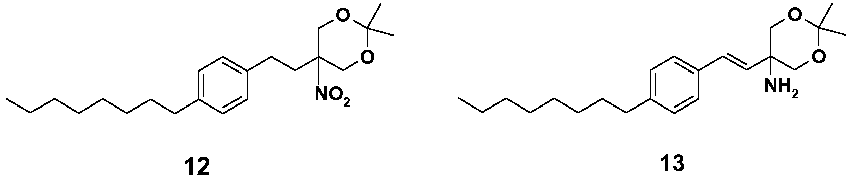

The intermediates (12) and (13) due to partial reduction were formed during the course of the reaction and were characterized by ¹H NMR, and ¹³C NMR. It was concluded based on the structures for (12) and (13) that partial reduction of compound (10), either at the double bond or that of nitro group respectively gave intermediates (12) and (13), which were subsequently reduced to give 1-(4-(2-(2,2-dimethyl-5-amino-1,3-dioxane-5-yl)-ethyl)phenyl)octane (11).

1-(4-(2-(2,2-Dimethyl-5-amino-1,3-dioxane-5-yl)-ethyl)phenyl)octane (11) was treated with an acid in an alcohol or ester solvent at 25-30°C to give fingolimod (I), which was then converted to the corresponding hydrochloride salt.

The acid used was either anhydrous or in the form of an aqueous solution. The aqueous acid solution had strength of 2% to 15 %, preferably in the range of 8 to 10%.

The acid was selected from the group comprising of hydrochloric acid, sulfuric acid, hydrobromic acid, acetic acid, trifluro acetic acid (TFA), p-toluene sulfonic acid (PTSA).

Fingolimod or its salts so obtained had desired purity and the associated impurities were well within the limits set by ICH guidelines.

The advantages of this novel route of synthesis for fingolimod are listed below.
a) By utilizing nitro acetonide of formula (7), impurities such as the nitroso [O-alkyl (R-O-NO)] or the nitration impurities arising from the generalized nitration reaction using metal nitrites as reported in the prior art references were avoided thereby resulting in substantial increase in yield. The method thus avoided purification steps for removal of these impurities. This step of the present invention also leads to novel intermediates, compounds (8), (9) and (10).
b) The novel intermediate (10), upon reduction leads to protected compound (11), which is the penultimate stage in present strategy of fingolimod synthesis. Thus, the protection of amino group as disclosed in the applications, WO 2012056458 and WO2012100399 is avoided. This subsequently eliminates further deprotection step with the accompanying unit operations and the associated increase in cost and batch time-cycle, thereby making the present process shorter, economical and efficient as compared to prior art processes.
c) The key intermediates of this process, viz. compounds (8), (9) and (10) are obtained as solids with high purity as compared to an oil or a sticky mass encountered in prior art. Further, the convenience in handling and transfer of the intermediates, provided an easy tool to obtain good control over the level of impurities such as the compounds of formula (2), (3) and (5) formed in the respective reactions. When the impurities are thus controlled at intermediate stages, the task of achieving the desired impurity profile for the final compound is made easy, thus avoiding extensive purification procedures at the final stage.
d) All the reactions are carried out at moderate temperatures and do not involve harsh conditions or lengthy reaction time. This further controls formation of undesired impurities and aids in the ease of operation and overall yield.

Thus, based on the above advantages, the present invention satisfies the need for developing an alternate synthetic route for fingolimod which has lesser number of synthetic steps, which is cost effective, environment-friendly and yet can be easily implemented on industrial scale.

The present invention is described herein below with reference to examples.

### EXAMPLES:

### Example 1: Preparation of 2-(4-octylphenyl)-acetaldehyde (6)

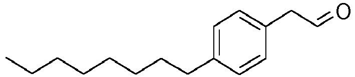

Dess-Martin periodinane (DMP; 199.3 g) was added with stirring to the mixture of 2-(4-octylphenyl)ethanol (5; 100.1 g) in dichloromethane (1000 ml) at 0°C to 5°C and stirred at the same temperature till completion of reaction, as monitored by TLC. The reaction mass was filtered and the filtrate was washed with bicarbonate solution and concentrated. Cyclohexane (1000ml) was added with stirring to the reaction mixture and filtered. The filtrate was concentrated to give 2-(4-octylphenyl)-acetaldehyde (6).
Yield: 83.2 g (83.7 %).

### Example 2: Preparation of 1-(4-(2-hydroxy-2-(2,2-dimethyl-5-nitro-1,3-dioxane-5-yl)-ethyl)phenyl)octane (8)

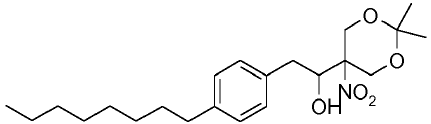

5-nitro-2,3-dimethyl-1,3-dioxane (**7;** 50.1gms) was added to a stirred mixture of 2-(4-octylphenyl) acetaldehyde (6; 80.6gms) and diisopropylethylamine (66.7gms) in dichloromethane (80 ml) at 25 to 35°C and stirred till completion of reaction as monitored by TLC. The organic layer was separated and concentrated. Cyclohexane (800 ml) was added to the residue and the stirred mass was filtered. Resulting solid was dried to yield compound (8).
Yield: 82.5 g, (67 %).
**¹H NMR (CDCl₃, 400 MHz, δ ppm):** 7.15 (d, J = 8.0 Hz, 2H), 7.10 (d, J = 8.0 Hz, 2H), 4.54 (m, 2H), 4.27 (d, J = 13.2 Hz, 1H), 4.19 (d, J = 13.2 Hz, 1H), 4.13- 4.10 (m, 1H), 2.85 (dd, J= 14.0, 2.3 Hz, 1H), 2.59-2.53 (m, 3H), 2.03 (d, J = 4.9 Hz, 1H), 1.60- 1.57 (m, 2H), 1.45 (s, 3H), 1.40 (s, 3H), 1.30- 1.27 (m, 10H), 0.88 (t, J = 6.5 Hz, 3H).
**¹³C NMR (CDCl₃, 100 MHz, δ ppm):** 142.0, 133.3, 129.1, 128.9, 99.3, 89.7, 73.4, 61.5, 61.3, 37.5, 35.5, 31.8, 31.4, 29.4, 29.3, 29.2, 25.7, 22.6, 20.8, 14.0.
**ESI-Mass:** For C₂₂H₃₅NO₅ (M⁺)/z: 393.53, Found: (M⁺H)/z: 394.3, (M⁺Na)/z: 416.2.
**Melting Point:** 83-86°C

### Example 3: Preparation of 1-(4-(2-methanesulfonyl-2-(2,2-dimethyl-5-nitro-1,3-dioxane-5-yl)-ethyl)phenyl)octane (9a)

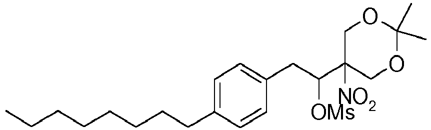

Methane sulfonyl chloride (34.8 g) was added to a mixture of 1-(4-(2-hydroxy-2-(2,2-dimethyl-5-nitro-1,3-dioxane-5-yl)-ethyl)phenyl)octane (8; 80.4gms) and triethylamine (51.5gms) in dichloromethane (400 ml) at 0 to 5°C, and the reaction mixture was stirred at 20-40°C. After completion of reaction as monitored by TLC, water was added to the reaction mixture and the layers were separated. The organic layer was concentrated to give 1-(4-(2-methanesulfonyl-2-(2,2-dimethyl-5-nitro-1,3-dioxane-5-yl)-ethyl)phenyl)octane (9a).
Yield: 83 g, (86 %).
**¹H NMR (CDCl₃, 400 MHz, δ ppm) :** 7.17-7.12 (m, 4H), 5.21 (dd, J = 10.5, 3.5 Hz, 1H), 4.60-4.51 (m, 2H), 4.25-4.17 (m, 2H), 3.01 (dd, J = 14.6, 3.6 Hz, 1H), 2.87-2.81 (m, 1H), 2.57 (t, J = 7.6 Hz, 2H), 2.2 (s, 3H), 1.57-1.53 (m, 2H), 1.46 (s, 3H), 1.39 (s, 3H), 1.27-1.25 (m, 10H), 0.87 (t, J = 6.44 Hz, 3H).
**¹³C NMR (CDCl₃, 100 MHz, δ ppm):** 142.8, 131.8, 129.6, 129.0, 99.4, 88.0, 81.4, 62.0, 61.2, 38.0, 35.5, 35.4, 31.8, 31.4, 29.3, 29.2, 29.1, 25.8, 22.6, 20.6, 14.0.
**ESI-Mass:** For C₂₃H₃₇NO₇S (M⁺)/z: 471.62, Found: (M⁺H)/z: 472.3, (M⁺Na)/z: 494.2. **Melting Point:** 62-65°C

### Example 4: Preparation of 1-(4-(2-(2,2-dimethyl-5-nitro-1,3-dioxane-5-yl)-ethene-1-yl) phenyl)octane (10)

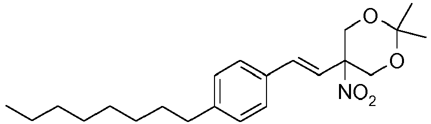

DBU (28.4gms) was added to a stirred solution of compound (9a; 80gms) in toluene (320 ml), and mixture heated to 80 to 90°C. After completion of the reaction as monitored by TLC, the reaction mass was cooled, diluted with toluene (480 ml) and washed with aq. HCl solution (800 ml). The organic layer was separated and concentrated to afford compound (10) as light brown solid.
Yield 60.5 g, (95 %)
**¹H NMR (CDCl₃, 400 MHz, δ ppm) :** 7.27 (d, J = 8.2 Hz, 2H), 7.15 (d, J = 8.1 Hz, 2H), 6.67 (d, J = 16.4 Hz, 1H), 6.0 (d, J = 16.4 Hz, 1H), 4.79 (dd, J = 12.0, 1.0 Hz, 2H), 4.15 (d, J = 13.0 Hz, 2H), 2.59 (t, J = 7.5 Hz, 2H), 1.59 (t, J = 7.3 Hz, 2H), 1.50 (s, 3H), 1.41 (s, 3H), 1.31-1.27 (m, 10H), 0.89 (t, J = 6.7 Hz, 3H).
**¹³C NMR (CDC13, 100 MHz, δ ppm):** 144.4, 135.0, 131.8, 128.8, 126.7, 119.5, 98.6, 85.8, 64.0, 35.6, 31.7, 31.1, 29.3, 29.1, 27.8, 22.5, 18.7, 14.0.
**ESI-Mass:** For C₂₂H₃₃NO₄ (M⁺)/z: 375.51, Found: (M⁺Na)/z: 398.2.
**Melting Point:** 117-119°C

### Example 5: Preparation of 1-(4-(2-(2,2-dimethyl-5-nitro-1,3-dioxane-5-yl)-ethene-1-yl) phenyl) octane (10)

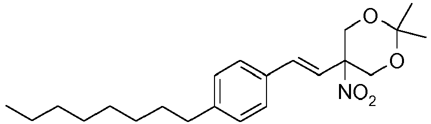

To a solution of compound (8; 10.3 g) in toluene (50 ml), p-toluene sulfonic acid (PTSA) (0.39 g) was added and mixture was heated to 80-100°C. After completion of the reaction as monitored by HPLC, the reaction mixture was cooled, diluted with toluene (50 ml). The toluene layer was concentrated to yield compound (10) as light brown solid.
Yield 6.7 g, (70 %)

### Example 6: Preparation of 1-(4-(2-(2,2-dimethyl-5-amino-1,3-dioxane-5-yl)-ethyl)phenyl)octane (11)

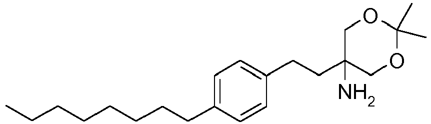

Compound (10; 60.5gms), ethyl acetate (720 ml) were charged to an autoclave and 10% Pd/C (6gms; on dry basis) was added to it. Hydrogen pressure of 7- 8 kg/cm² was applied and mixture was heated to 75 to 80 °C. After complete of the reaction as monitored by TLC, reaction mixture was cooled and catalyst was filtered. Filtrate was concentrated to give compound (11) as residue.
Yield 36.2 gms. (65%)
**¹H NMR (CDCl₃, 400 MHz, δ ppm):** 7.10 - 7.06 (m, 4H), 3.78 (d, J = 11.5 Hz, 2H), 3.46 (d, J = 11.5 Hz, 2H), 2.64- 2.60 (m, 2H), 2.55 (t, J = 7.6 Hz, 2H), 1.63 -1.54 (m, 4H), 1.44 (s, 3H), 1.41 (s, 3H), 1.29-1.26 (m, 10H), 0.87 (t, J = 6.7 Hz, 3H).
**¹³C NMR (CDCl₃, 100 MHz, δ ppm):** 140.5, 139.0, 128.4, 128.0, 98.1, 70.0, 48.6, 37.6, 35.4, 31.8, 31.5, 29.4, 29.3, 29.2, 28.2, 27.3, 22.6, 19.8, 14.0.
ESI-Mass: For C₂₂H₃₇NO₂ (M⁺)/z: 347.55, Found: (M⁺H)/z: 348.1, (M⁺Na)/z: 370.3.

### Example 7: Preparation of Fingolimod hydrochloride (1a)

Hydrochloric acid dissolved in ethyl acetate (53 ml, 10%) was added to a solution of compound (11; 35 g.) in ethyl acetate (105 ml), and reaction mixture was stirred at 25-30°C. After completion of the reaction, as monitored by TLC, the mass was concentrated. The resulting residue was stirred in ethyl acetate (105 ml) at 0 to 10°C and filtered to obtain fingolimod hydrochloride (1a) as white solid.
Yield 26.3 g, (76 %)

## Claims

1. A process for preparation of fingolimod hydrochloride comprising,
a) reacting 2-(4-octylphenyl)-ethanol with an oxidizing agent, in an organic solvent to give 2-(4-octylphenyl)-acetaldehyde of formula (6), 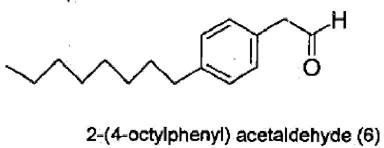
b) treating compound of formula (6) with 5-nitro-2,3-dimethyl-1,3-dioxane of formula (7) 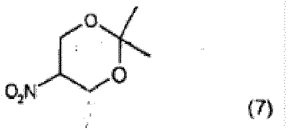 in presence of a base to give 1-(4-(2-hydroxy-2-(2,2-dimethyl-5-nitro-1,3-dioxane-5-yl)ethyl) phenyl) octane of formula (8), 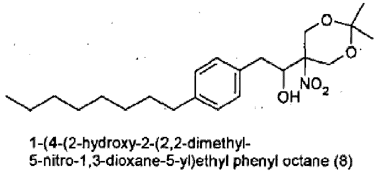
c) treating compound of formula (8) with substituted sulfonyl chloride in presence of triethylamine to give the sulfonates of compound (9), 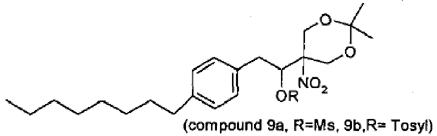
d) treating compound of formula (9) with a base to give 1-(4-(2-(2,2-dimethyl-5-nitro-1,3-dioxane-5-yl)-ethene-1-yl)phenyl)octane of formula (10), 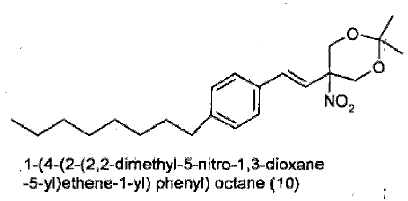 which on hydrogenation gives 1-(4-(2-(2,2-dimethyl-5-amino-1,3-dioxane-5-yl)- ethyl)phenyl)octane of formula (11), 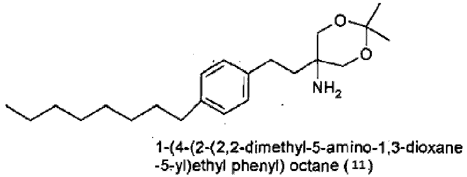
e) treating compound of formula (11) with an acid to yield fingolimod (I) which is further converted to its hydrochloride salt.

2. A process for preparation of fingolimod hydrochloride comprising,
a) reacting 2-(4-octylphenyl)-ethanol with an oxidizing agent, in an organic solvent to give 2-(4-octylphenyl)-acetaldehyde of formula (6), 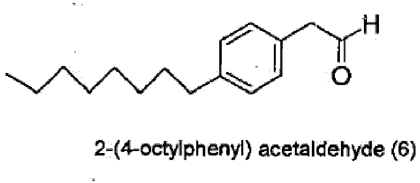
b) treating compound of formula (6) with 5-nitro-2,3-dimethyl-1,3-dioxane of formula (7) 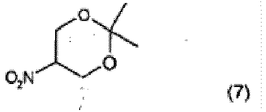 in presence of a base to give 1-(4-(2-hydroxy-2-(2,2-dimethyl-5-nitro-1,3-dioxane-5-yl)-ethyl) phenyl) octane of formula (8), 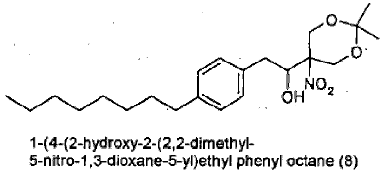
c) treating compound of formula (8) with a dehydrating agent to give 1-(4-(2-(2,2-dimethyl-5-nitro-1,3-dioxane-5-yl)-ethene-1-yl)phenyl)octane of formula (10), 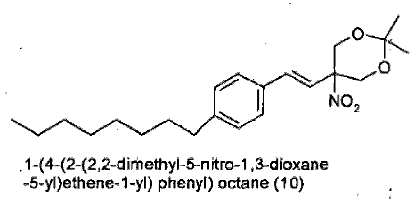
d) hydrogenating compound of formula (10) to give 1-(4-(2-(2,2-dimethyl-5-amino-1,3-dioxane-5-yl)- ethyl)phenyl)octane of formula (11), 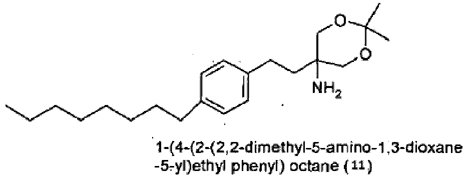
e) treating compound of formula (11) with an acid to yield fingolimod (I) which is further converted to its hydrochloride salt.

3. The process as claimed in step a) of claims 1 and 2, wherein the oxidizing agent is selected from Dess-Martin periodinane (DMP), (2,2,6,6-tetramethylpiperidin-1-yl)oxidanyl (TEMPO), sodium hypochlorite, pyridinium dichromate (PDC), pyridinium chlorochromate (PCC), dimethylsulfoxide/triethylamine/oxalyl chloride (DMSO/TEA/(COCl)₂ and sulfur trioxide /pyridine (SO₃-Py).

4. The process as claimed in step a) of claims 1 and 2, wherein the solvent is selected from the group comprising of esters, ethers, nitriles, halogenated hydrocarbons, and aromatic/aliphatic hydrocarbons.

5. The process as claimed in step b) of claim 1, step d) of claim 1 and step b) of claim 2, wherein base is selected from triethylamine (TEA), 1,8-Diazabicyclo[5.4.0]undec-7-ene (DBU), 1,5-Diazabicyclo(4.3.0)non-5-ene (DBN), 1,4-diazabicyclo[2.2.2]octane (DABCO), 2,6-dimethylpyridine (2,6-lutidine), diisopropylethylamiine (DIPEA), N-methyl morpholine (NMM), pyridine, N,N-dimethylaminopyridine (DMAP) and mixtures thereof.

6. The process as claimed in step c) of claims 1, wherein the substituted sulfonyl chloride is selected from a group comprising of methanesulfonyl chloride, p-toluene sulfonyl chloride.

7. The process as claimed in step c) of claim 2, wherein the dehydrating agent is selected from phosphorus pentoxide (P₂O₅), p-toluene sulfonic acid (PTSA) and mixtures thereof.

8. The process as claimed in step e) of claims 1 and 2, wherein the acid is selected from the group comprising of hydrochloric acid, sulfuric acid, hydrobromic acid, acetic acid, trifluro acetic acid (TFA) and p-toluene sulfonic acid (PTSA).

9. 1-(4-(2-Hydroxy-2-(2,2-dimethyl-5-nitro-1,3-dioxane-5-yl)-ethyl)phenyl) octane of formula (8).

10. 1-(4-(2-(2,2-Dimethyl-5-nitro-1,3-dioxane-5-yl)-ethene-1-yl)phenyl)-octane of formula (10).

## Patentansprüche

1. Verfahren zur Herstellung von Fingolimod Hydrochlorid, umfassend
a) Reagieren von 2-(4-Octylphenyl)ethanol mit einem Oxidationsmittel in einem organischen Lösungsmittel, um 2-(4-Octylphenyl)acetaldehyd der Formel (6) zu ergeben, 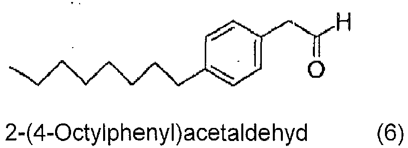
b) Behandeln der Verbindung der Formel (6) mit 5-Nitro-2,3-dimethyl-1,3-dioxan der Formel (7) 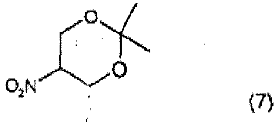 in Gegenwart einer Base, um 1-(4-(2-Hydroxy-2-(2,2-dimethyl-5-nitro-1,3-dioxan-5-yl)ethyl)phenyl)octan der Formel (8) zu ergeben, 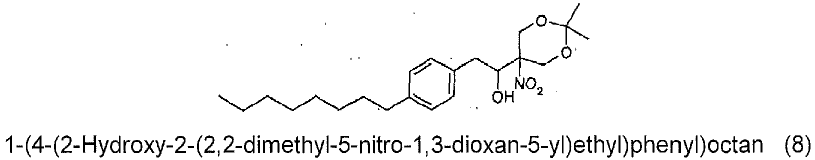
c) Behandeln der Verbindung der Formel (8) mit substituiertem Sulfonylchlorid in Gegenwart von Triethylamin, um die Sulfonate der Verbindung (9) zu ergeben, 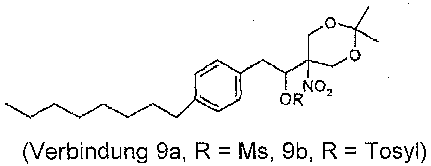
d) Behandeln der Verbindung der Formel (9) mit einer Base, um 1-(4-(2-(2,2-Dimethyl-5-nitro-1,3-dioxan-5-yl)-ethen-1-yl)phenyl)octan der Formel (10) zu ergeben, 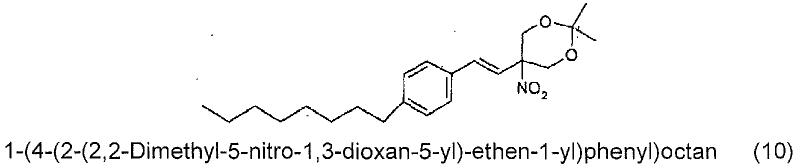 das bei Hydrierung 1-(4-(2-(2,2-Dimethyl-5-amino-1,3-dioxan-5-yl)-ethyl)phenyl)octan der Formel (11) ergibt, 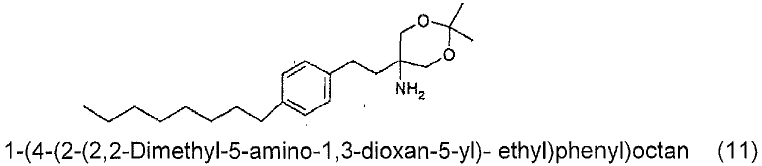
e) Behandeln der Verbindung der Formel (11) mit einer Säure, um Fingolimod (I) zu ergeben, das weiter zu seinem Hydrochlorid-Salz umgewandelt wird.

2. Verfahren zur Herstellung von Fingolimod-Hydrochlorid, umfassend
a) Reagieren von 2-(4-Octylphenyl)ethanol mit einem Oxidationsmittel in einem organischen Lösungsmittel, um 2-(4-Octylphenyl)acetaldehyd der Formel (6) zu ergeben, 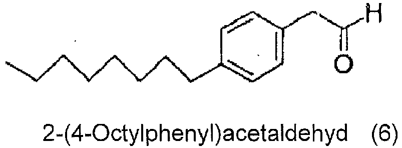
b) Behandeln der Verbindung der Formel (6) mit 5-Nitro-2,3-dimethyl-1,3-dioxan der Formel (7) 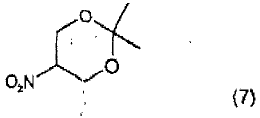 in Gegenwart einer Base, um 1-(4-(2-Hydroxy-2-(2,2-dimethyl-5-nitro-1,3-dioxan-5-yl)ethyl)phenyl)octan der Formel (8) zu ergeben, 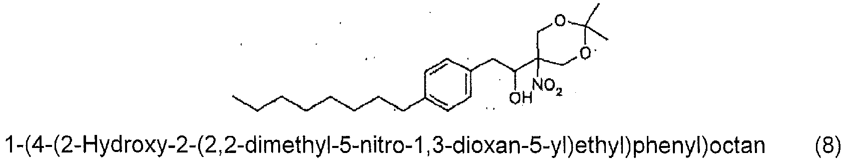
c) Behandeln der Verbindung der Formel (8) mit einem Dehydrierungsmittel, um 1-(4-(2-(2,2-Dimethyl-5-nitro-1,3-dioxan-5-yl)-ethen-1-yl)phenyl)octan der Formel (10) zu ergeben, 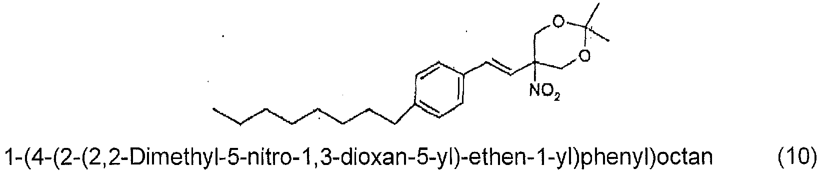
d) Hydrieren der Verbindung der Formel (10), um 1-(4-(2-(2,2-Dimethyl-5-amino-1,3-dioxan-5-yl)ethyl)phenyl)octan der Formel (11) zu ergeben, 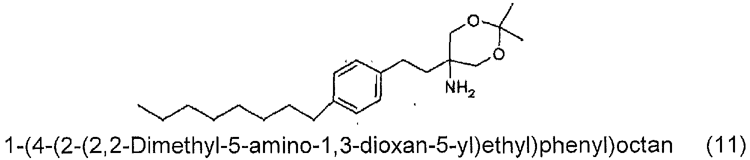
e) Behandeln der Verbindung der Formel (11) mit einer Säure, um Fingolimod (I) zu ergeben, das weiter zu seinem Hydrochlorid-Salz umgewandelt wird.

3. Verfahren, wie in Schritt a) der Ansprüche 1 und 2 beansprucht, wobei das Oxidationsmittel ausgewählt ist aus Dess-Martin Periodinan (DMP), (2,2,6,6-Tetramethylpiperidin-1-yl)oxidanyl (TEMPO), Natriumhypochlorit, Pyridiniumdichromat (PDC), Pyridiniumchlorochromat (PCC), Dimethylsulfoxid/Triethylamin/Oxalylchlorid (DMSO/TEA/(COCl)₂ und Schwefeltrioxid / Pyridin (SO₃-Py).

4. Verfahren, wie in Schritt a) der Ansprüche 1 und 2 beansprucht, wobei das Lösungsmittel ausgewählt ist aus der Gruppe, umfassend Ester, Ether, Nitrile, halogenierte Kohlenwasserstoffe und aromatische/aliphatische Kohlenwasserstoffe.

5. Verfahren, wie in Schritt b) des Anspruchs 1, Schritt d) des Anspruchs 1, und Schritt b) des Anspruchs 2 beansprucht, wobei die Base ausgewählt ist aus Triethylamin (TEA), 1,8-Diazabicyclo[5.4.0]undec-7-en (DBU), 1,5-Diazabicyclo(4.3.0)non-5-en (DBN), 1,4-Diazabicyclo[2.2.2]octan (DABCO), 2,6-Dimethylpyridin (2,6-Lutidin), Diisopropylethylamin (DIPEA), N-Methylmorpholin (NMM), Pyridin, N,N-Dimethylaminopyridin (DMAP), und Mischungen davon.

6. Verfahren, wie in Schritt c) des Anspruchs 1 beansprucht, wobei das substituierte Sulfonylchlorid ausgewählt ist aus der Gruppe, umfassend Methansulfonylchlorid, p-Toluolsulfonylchlorid.

7. Verfahren, wie in Schritt c) des Anspruchs 2 beansprucht, wobei das Dehydrierungsmittel ausgewählt ist aus Phosphorpentoxid (P₂O₅), p-Toluolsulfonsäure (PTSA), und Mischungen davon.

8. Verfahren, wie in Schritt e) der Ansprüche 1 und 2 beansprucht, wobei die Säure ausgewählt ist aus der Gruppe, umfassend Salzsäure, Schwefelsäure, Bromwasserstoffsäure, Essigsäure, Trifluoressigsäure (TFA) und p-Toluolsulfonsäure (PTSA).

9. 1-(4-(2-Hydroxy-2-(2,2-dimethyl-5-nitro-1,3-dioxan-5-yl)ethyl)phenyl)octan der Formel (8).

10. 1-(4-(2-(2,2-Dimethyl-5-nitro-1,3-dioxan-5-yl)-ethen-1-yl)phenyl)octan der Formel (10).

## Revendications

1. Procédé de préparation du chlorhydrate de fingolimod comprenant,
a) la réaction du 2-(4-octylphényl)-éthanol avec un agent oxydant, dans un solvant organique pour donner le 2-(4-octylphényl)-acétaldéhyde de formule (6), 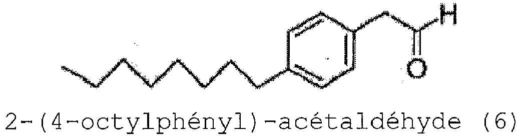
b) le traitement du composé de formule (6) avec le 5-nitro-2,3-diméthyl-1,3-dioxane de formule (7) 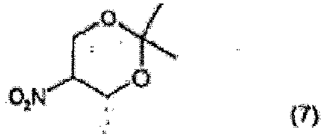 en présence d'une base pour donner le 1-(4-(2-hydroxy-2-(2,2-diméthyl-5-nitro-1,3-dioxan-5-yl)éthyl)-phényl)octane de formule (8), 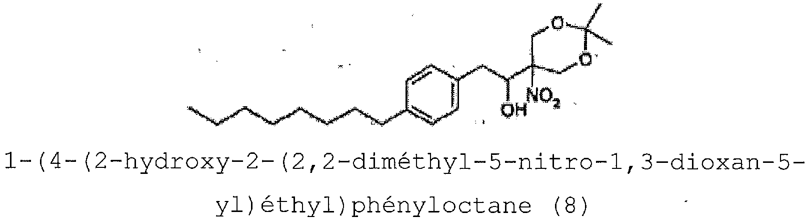
c) le traitement du composé de formule (8) avec un chlorure de sulfonyle substitué en présence de triéthylamine pour donner les sulfonates de composé (9), 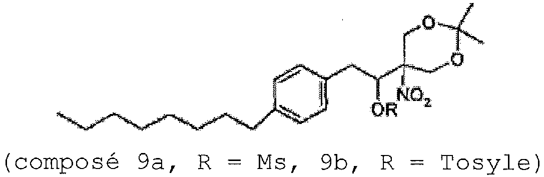
d) le traitement du composé de formule (9) avec une base pour donner le 1-(4-(2-(2,2-diméthyl-5-nitro-1,3-dioxan-5-yl)-éthèn-1-yl)phényl)octane de formule (10), 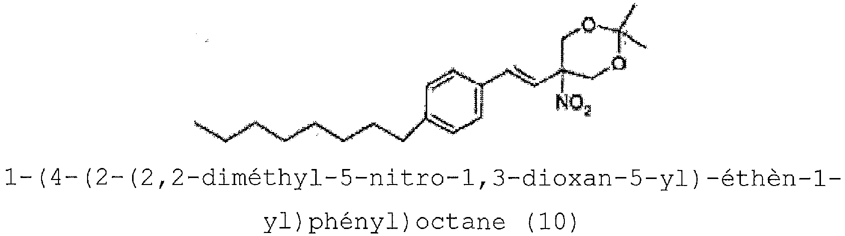 qui donne après hydrogénation le 1-(4-(2-(2,2-diméthyl-5-amino-1,3-dioxan-5-yl)éthyl)phényl)octane de formule (11), 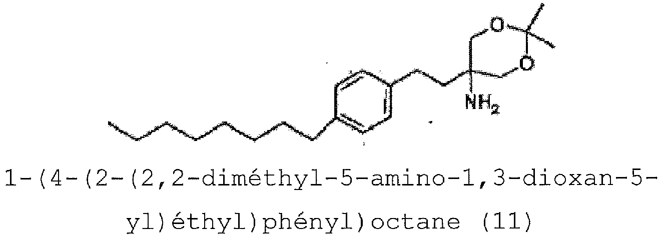
e) le traitement du composé de formule (11) avec un acide pour donner le fingolimod (I) qui est converti ensuite en son sel de chlorhydrate.

2. Procédé de préparation du chlorhydrate de fingolimod comprenant,
a) la réaction du 2-(4-octylphényl)-éthanol avec un agent oxydant, dans un solvant organique pour donner le 2-(4-octylphényl)-acétaldéhyde de formule (6), 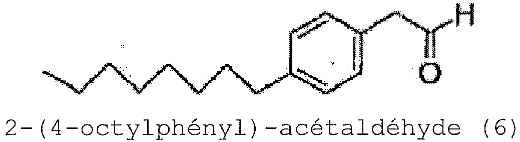
b) le traitement du composé de formule (6) avec le 5-nitro-2,3-diméthyl-1,3-dioxane de formule (7) 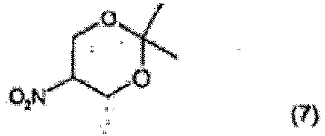 en présence d'une base pour donner le 1-(4-(2-hydroxy-2-(2,2-diméthyl-5-nitro-1,3-dioxan-5-yl)éthyl)-phényl)octane de formule (8), 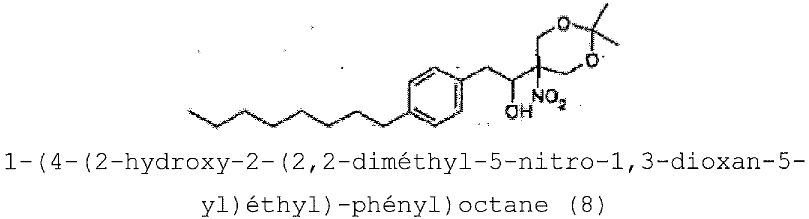
c) le traitement du composé de formule (8) avec un agent de déshydratation pour donner le 1-(4-(2-(2,2-diméthyl-5-nitro-1,3-dioxan-5-yl)-éthèn-1-yl)phényl)-octane de formule (10), 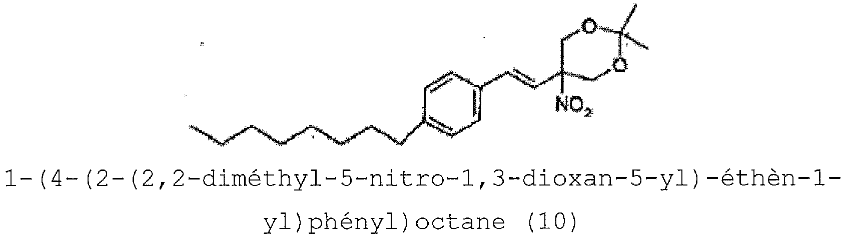
d) l'hydrogénation du composé de formule (10) pour donner le 1-(4-(2-(2,2-diméthyl-5-amino-1,3-dioxan-5-yl)éthyl)phényl)octane de formule (11), 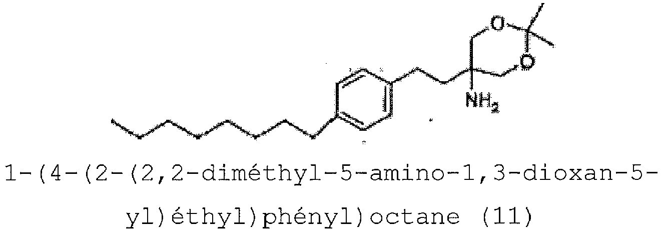
e) le traitement du composé de formule (11) avec un acide pour donner le fingolimod (I) qui est converti ensuite en son sel de chlorhydrate.

3. Procédé selon l'étape a) des revendications 1 et 2, dans lequel l'agent oxydant est sélectionné parmi le periodinane de Dess-Martin (DMP), le (2,2,6,6-tétraméthylpipéridin-1-yl)oxidanyle (TEMPO), l'hypochlorite de sodium, le dichromate de pyridinium (PDC), le chlorochromate de pyridinium (PCC), le diméthylsulfoxyde/triéthylamine/chlorure d'oxalyle (DMSO/TEA/(COCl)₂ et le trioxyde de soufre/pyridine (SO₃-Py).

4. Procédé selon l'étape a) des revendications 1 et 2, dans lequel le solvant est sélectionné dans le groupe comprenant les esters, les éthers, les nitriles, les hydrocarbures halogénés, et les hydrocarbures aromatiques/aliphatiques.

5. Procédé selon l'étape b) de la revendication 1, l'étape d) de la revendication 1 et l'étape b) de la revendication 2, dans lequel la base est sélectionnée parmi la triéthylamine (TEA), le 1,8-diazabicyclo [5.4.0]undéc-7-ène (DBU), le 1,5-diazabicyclo(4.3.0) non-5-ène (DBN), le 1,4-diazabicyclo[2.2.2]octane (DABCO), la 2,6-diméthylpyridine (2,6-lutidine), la diisopropyléthylamine (DIPEA), la N-méthyl morpholine (NMM), la pyridine, la N,N-diméthylaminopyridine (DMAP) et leurs mélanges.

6. Procédé selon l'étape c) de la revendication 1, dans lequel le chlorure de sulfonyle substitué est sélectionné dans un groupe comprenant le chlorure de méthanesulfonyle, le chlorure de p-toluènesulfonyle.

7. Procédé selon l'étape c) de la revendication 2, dans lequel l'agent de déshydratation est sélectionné parmi le pentoxyde de phosphore (P₂O₅), l'acide p-toluènesulfonique (PTSA) et leurs mélanges.

8. Procédé selon l'étape e) des revendications 1 et 2, dans lequel l'acide est sélectionné dans le groupe comprenant l'acide chlorhydrique, l'acide sulfurique, l'acide bromhydrique, l'acide acétique, l'acide trifluoroacétique (TFA) et l'acide p-toluène sulfonique (PTSA).

9. 1-(4-(2-Hydroxy-2-(2,2-diméthyl-5-nitro-1,3-dioxan-5-yl)-éthyl)phényl)octane de formule (8).

10. 1-(4-(2-(2,2-Diméthyl-5-nitro-1,3-dioxan-5-yl)-éthèn-1-yl)phényl)octane de formule (10).
